# EUROPEAN PATENT APPLICATION

(11) **EP 2 573 552 A1**
(43) Date of publication of application: **27.03.2013**
(21) Application number: 11783426.7
(22) Date of filing: 11.05.2011
(51) Int. Cl.: G01N 27/16

(54) **CATALYTIC COMBUSTION TYPE GAS SENSOR**

(30) Priority: 17.05.2010 JP 2010112973
(71) Applicant: Honda Motor Co., Ltd., Minato-ku Tokyo 107-8556 (JP)
(72) Inventor: OKAJIMA Kazuhiro, Wako-shi Saitama 351-0193 (JP); OISHI Hidetoshi, Wako-shi Saitama 351-0193 (JP); TSUKABAYASHI Shunji, Wako-shi Saitama 351-0193 (JP); SUZUKI Akihiro, Wako-shi Saitama 351-0193 (JP); MURAKAMI Nobuaki, Itami-shi Hyogo 664-0891 (JP); MORIMOTO Satoshi, Itami-shi Hyogo 664-0891 (JP); KODA Hiroshi, Itami-shi Hyogo 664-0891 (JP); MATSUMOTO Takashi, Itami-shi Hyogo 664-0891 (JP)
(74) Representative: Herzog, Markus
(86) International application number: PCT/JP2011/060825
(87) International publication number: WO 2011/145492

(57) **Abstract**

Provided is a catalytic combustion type gas sensor in which the temperature and the electrical resistance of a catalyst metal are increased by combustion heat generated when a combustible gas burns upon contact with the catalyst metal that has been heated by voltage application, and in which the combustible gas present in a given or higher concentration is detected based on the increase in electrical resistance. In the sensor, a voltage is applied to the catalyst metal (detection element) so that the catalyst metal has a standby temperature, which is obtained by subtracting the temperature increase caused by combustion heat generated when the combustible gas present in a given concentration burns upon contact with the catalyst metal (detection element) from a desorption temperature at which an adsorbate that has been adsorbed onto the catalyst metal through silicon (Si) poisoning is desorbed. The desorption temperature is set to a temperature in the range of 350-600°C, excluding 350°C. When the sensor is switched on and/or switched off, a voltage is applied to the catalyst metal (detection element) so that the catalyst metal (detection element) is heated to a temperature in the range of 350-600°C. Thus, the catalytic combustion type gas sensor can be inhibited from deteriorating in detection sensitivity.

## Description

### FIELD OF THE INVENTION

The present invention relates to a catalytic combustion typed gas sensor to be used for detecting a combustible gas.

### BACKGROUND OF THE INVENTION

Recently, much attention has been paid on a fuel cell using a combustible gas like hydrogen as a fuel of a clean energy source. Hereby, a fuel cell vehicle equipped with a fuel cell as the energy source is being developed for driving a vehicle. Therefore, a fuel cell vehicle is equipped with a gas sensor for detecting leakage of a combustible gas, assuming the case that such a combustible gas like hydrogen happens to leak.

Here, a catalytic typed gas sensor having a simple structure and being easily downsized is generally used as a gas sensor. It is known that as for such a catalytic typed gas sensor, if a vapor of a silicone compound is contained in the atmosphere of the operating environment, detection sensitivity of the sensor becomes deteriorated with times (that is, via silicon (Si) poisoning).
Therefore, a conventional catalytic typed gas sensor directly covers a detection element, which is poisoned by silicon, with a silicon trap layer (for example, see Patent Document 1) .

### PRIOR ART DOCUMENTS

### PATENT LITERATURES

Patent Document 1: International Patent Publication No. WO 2007/099933 pamphlet.

### DISCLOSURE OF THE INVENTION

### PROBLEMS TO BE SOLVED BY THE INVENTION

A conventional catalytic typed gas sensor tends to make a silicone compound contained in the atmosphere only adhere to a silicon trap layer. Thus, it has been thought that an adhesion amount of the silicone compound has limitations. Hereby, it has been further thought that although a period until the detection sensitivity becomes deteriorated may be elongated, the actual detection sensitivity of the sensor eventually can not be prevented from being deteriorated.
Further, a detection element is covered with a silicon trap layer, which results in the increase in the heat capacity of the detection sensor, thereby to hardly make the temperature of the detection element be raised. In this way, it has been considered that the detection sensitivity eventually becomes deteriorated.

### MEANS FOR SOLVING THE PROBLEMS

For solving the drawback as mentioned above, an object of the present invention is to provide a catalytic type of a gas sensor capable of suppressing the deterioration of the detection sensor.

According to the present invention, heat generation by combustion of a combustible gas when the combustible gas contacts to a catalytic metal that is heated by passing electric current through the catalytic metal raises the temperature and the electric resistance of the catalytic metal. That resulting raised electric resistance allows the catalytic combustion typed gas sensor to detect the combustible gas having a concentration equal to or more than a predetermined gas concentration.
Herein, the electric current is made to pass through the catalytic metal such that the standby temperature is set at the temperature calculated by subtracting the resulting raised temperature portion from the desorption temperature. Herein, the resulting raised temperature portion is generated by the combustion heat when the combustible gas having the predetermined concentration contacts to the catalytic metal. The catalytic metal adsorbs a silicone compound via a silicon poisoning process and desorbs the adsorbed silicone compound at the desorption temperature.

The above mentioned procedure enables the detection element to be constructed by using the catalytic metal. Further, the present inventors have demonstrated that a desorption temperature exists at which an adsorbate (or silicone compound) adsorbed through the silicon poisoning process to the catalytic metal (or detection element) is to be desorbed from the detection element. Moreover, the present inventors have demonstrated that the desorption temperature is in the range from 350°C to 600°C. Within the range of the desorption temperature, the higher the temperature is, the easier the detection sensitivity is recovered. When the temperature of the catalytic metal (or detection element) reaches the desorption temperature, the adsorbate (or silicone compound) is desorbed, allowing the deteriorated detection sensitivity to be recovered.

Furthermore, the standby temperature is set at the temperature calculated by subtracting the raised temperature portion from the desorption temperature. Herein, the raised temperature portion is generated by the combustion heat when the combustible gas having the predetermined concentration contacts to the catalytic metal (or detection element). Accordingly, whenever the concentration of the combustion gas in the atmosphere reaches the predetermined concentration, a temperature of the catalytic metal (or detection element) may reach the desorption temperature. This allows the detection sensitivity of the catalytic combustion typed gas sensor to be recovered. Further, such a recovery allows the detection sensitivity to be prevented from being deteriorated.

Further, according to the present invention, it is preferable to set the desorption temperature in the range from more than 350°C to 600°C or less.

This temperature setting allows the detection sensitivity to be recovered whenever the concentration of the combustible gas in the atmosphere reaches the predetermined concentration for detecting the combustible gas.

Further, in the present invention, it is preferable to arrange the catalytic combustion typed gas sensor in the atmosphere in which the concentration of the silicone compound is higher than in the air. Moreover, it is also preferable to set the standby temperature in the range from substantially 100°C or more to substantially 350°C or less.

According to the above mentioned construction, the catalytic combustion typed gas sensor is arranged in the atmosphere having a higher concentration of the silicone compound than in the air. This allows the catalytic combustion typed gas sensor to be arranged inside an off-gas pipe from which an off-gas is discharged; the off-gas flowing from a fuel cell of a fuel cell vehicle. The reason is based on the fact that not a small amount of a silicone compound is used in a fuel cell. The predetermined concentration for detecting the combustible gas is set at a high concentration inside the off-gas pipe. Such setting of the high concentration increases the raised temperature portion to 250°C at the detection timing, allowing the standby temperature to be lowered to 350°C, even though the temperature reaches 600°C at the detection timing. Similarly, the setting of the high concentration enables the standby temperature to be lowered to 100°C, even though the temperature reaches 350°C at the detection timing.

Moreover, in the present invention, it is preferable to arrange the catalytic combustion typed gas sensor in the air and set the standby temperature in the range from substantially 270°C or more to substantially 520°C or less.

According to the above mentioned conditions, since the catalytic combustion typed gas sensor is arranged in the air, similarly the catalytic combustion typed gas sensor may be also arranged, for example, at the surroundings of a fuel cell and a hydrogen tank, disposed under a floor panel of a fuel cell vehicle or inside a cabin thereof. This may be explained by the fact that the silicone compound is not used more remarkably at the areas under a floor panel and inside a cabin than at other areas.
Further, the predetermined concentration for detecting a combustible gas is set at a low concentration, under a floor panel and inside a cabin. If the predetermined concentration is set at a low concentration, a raised temperature portion at the detection timing is lowered to 80°C. This allows the standby temperature to be raised up to 520°C at the detection timing, even though the temperature reaches 600°C at the detection timing, and similarly the standby temperature to be raised up to 270°C at the detection timing, even though the temperature reaches 350°C at the detection timing.

Further, in the present invention, it is preferable to pass electric current through the catalytic metal such that a temperature of the catalytic metal is set in the range from 350°C to 600C° at least at either of a startup period and a shutdown period.

The procedure enables the detection sensitivity to be recovered whenever the catalytic combustion typed gas sensor starts or stops the operation.

Further, in the present invention, it is preferable to arrange the catalytic combustion typed gas sensor in an off-gas discharge pipe from which the air supplied to a cathode of a fuel cell is discharged.

The construction enables hydrogen included in the off-gas discharge pipe to be detected.

Further, in the present invention, it is preferable to equip a fuel cell vehicle with the catalytic combustion typed gas sensor.

This construction enables the detection of hydrogen leaked into a fuel cell vehicle.

### ADVANTAGEOUS EFFECTS OF THE INVENTION

According to the present invention, provided is a catalytic combustion typed gas sensor which prevents the detection sensitivity of the sensor from being deteriorated.

### BRIEF DESCRIPTION OF THE DRAWINGS

FIG.1 is a schematic drawing showing a fuel cell vehicle equipped with a catalytic combustion typed gas sensor in an embodiment of the present invention.
FIG.2 is a cross-sectional drawing showing the catalytic combustion typed gas sensor in the embodiment of the present invention.
FIG.3A is a front view of a detection element having a coil shape.
FIG.3B is a cross sectional drawing of a detection element having a thin film shape.
FIG.4A is a circuit for passing electric current through elements, in the circuit a detection element and a compensation element are arrange in series.
FIG.4B is a circuit for passing electric current through elements. In the circuit, a detection element and a compensation element are arranged in parallel.
Fig.5 is a graphic diagram showing a relationship between a temperature of the detection element and an adhesive amount of the silicone compound.
FIG.6 is a graphic diagram showing a relationship between a concentration of the combustible gas and a temperature of the detection element. The diagram shows a method in detail for setting a desorption temperature and a standby temperature in the case that the detection concentration is set at 1.0% or more (vol%, such a dimension is the same hereinafter), and in the case that the detection concentration is set at 3.0% or more.

### MODES FOR CARRYING OUT THE INVENTION

Next, embodiments of the present invention will be explained in detail referring to the attached drawings. Note the same reference numerals are used for the common parts to omit the duplicated explanations.

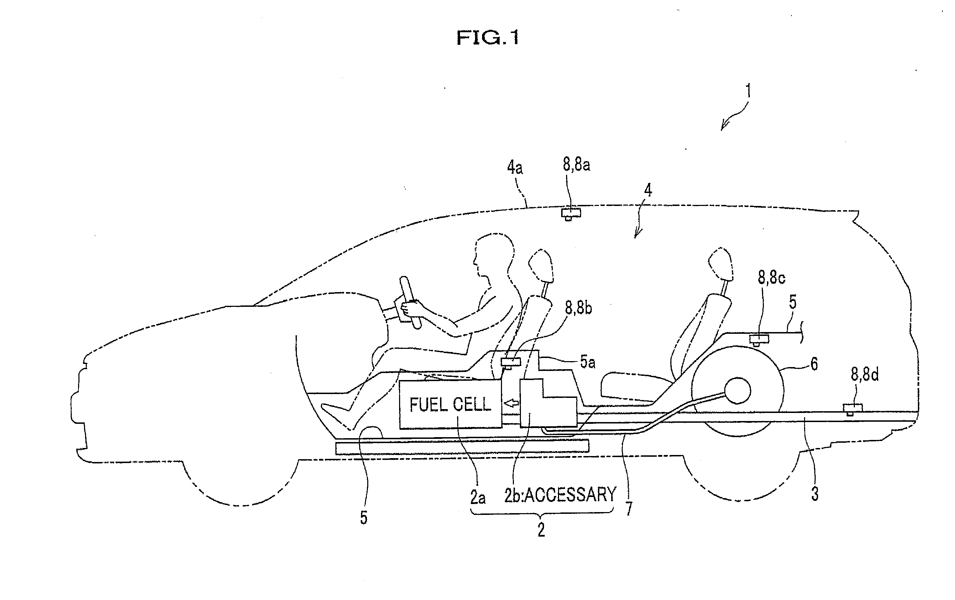

FIG.1 shows a schematic drawing of a fuel cell vehicle 1 equipped with a catalytic combustion typed gas sensor 8 in an embodiment of the present invention. The fuel cell vehicle 1 is equipped with a fuel cell system 2 and a hydrogen tank 6. The fuel cell system 2 comprises a fuel cell 2a generating electric power and an accessory 2b controlling the amount of the generated electric power. The fuel cell 2a and the accessory 2b are housed in a center console 5a which is constructed such that a part of the floor panel 5 rises. Further, the floor panel 5 rises at the back side of the fuel cell vehicle 1 such that the floor panel 5 covers an upper portion of the hydrogen tank 6.

The fuel cell 2a comprises, for example, an electrolyte film made of a solid polymer, electrode catalytic layers (that is, anode and cathode), and an MEA (Membrane Electrode Assembly) formed by stacking gas diffusion layers. Further, the fuel cell 2a has a structure formed by stacking a plurality of single cells, each of which is made by holding the both surfaces of the membrane electrode assembly with conductive separators, in a thickness direction of the cell (that is, in a longitudinal direction of the vehicle of the present embodiment). Further, passages are formed respectively in the separator facing against the anode and the cathode. Herein, hydrogen flows into one passage of the separator for the anode, and the air flows into the other passage of the separator for the cathode. Moreover, a through hole or the like is also formed for connecting the separators each other. The electrolyte membrane has a backbone made of a silicone resin containing silicon (Si) (that is, silicone compound).

Here, in such a fuel cell 2a, hydrogen supplied via a hydrogen supply pipe 7 from the hydrogen tank 6 to the separator at the anode side is diffused by a gas diffusion layer to be supplied to the anode. Similarly, the air (or oxygen) supplied from an air compressor to the separator at the cathode side is diffused by the gas diffusion layer to be supplied to the cathode. In the anode, hydrogen is separated into a hydrogen ion and an electron by the catalytic reaction, whereby the hydrogen ion reaches the cathode through the electrolyte membranes. Then, in the cathode, the hydrogen ion reaching therein through the membrane by the catalytic reaction, the electron transferred to the cathode through the outside load, and oxygen in the supplied air generate water via the electrochemical reaction. The air supplied from the air compressor includes the generated water, resulting in being discharged outside through an off-gas discharge pipe 3.

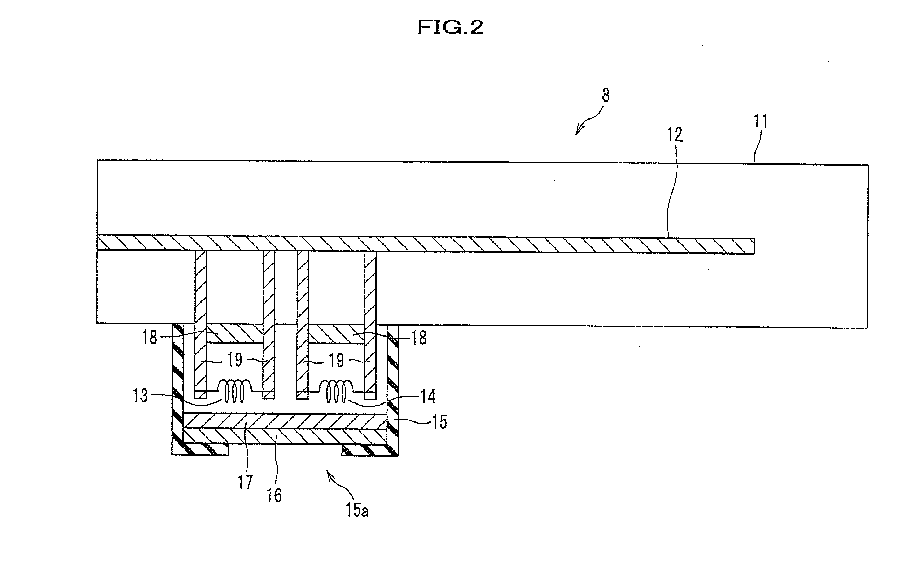

Since hydrogen is a combustible gas, the fuel cell vehicle 1 is equipped with a plurality of catalytic combustion typed gas sensors 8 such that leakage of the hydrogen may be detected if the leakage of the hydrogen occurs. The catalytic combustion typed gas sensor 8a (or 8) is arranged on a cabin top 4a in the cabin 4. If hydrogen leaks to inside the cabin 4, the hydrogen stays near the cabin top 4a since hydrogen is lighter than the air.
Further, the catalytic combustion typed gas sensor 8b (or 8) is arranged directly under the most upper portion of a center console 5a over the fuel cell 2a and the accessory 2b. The catalytic combustion typed gas sensor 8c (or 8) is arranged over the hydrogen tank 6 and directly below the floor panel 5. The catalytic combustion typed gas sensor 8d (or 8) is arranged such that a detection unit 15 (or detection element 13) described hereinafter (see FIG.2) is inserted to the inside of the off-gas discharge pipe 3 so as to detect hydrogen included inside the off-gas discharge pipe 3.

The atmosphere in which the catalytic combustion typed gas sensors 8a, 8b and 8c detect the leaked hydrogen after the hydrogen has been diffused into the air to have a low concentration. Accordingly, the gas sensors are set up such that the sensors are capable of detecting the leaked hydrogen at a low concentration. The atmosphere in which the catalytic combustion typed gas sensors 8a, 8b and 8c are placed is identical to the air. Further, the concentration of the silicone compound in the atmosphere is almost the same as the concentration of the silicone compound in the air. Herein, those concentrations are assumed to be low.

The leaked hydrogen inside the fuel cell 2a and the accessory 2b is not diffused in the air and flows into the inside of the off-gas discharge pipe 3. Therefore, the catalytic combustion typed gas sensor 8d is set such that the gas sensor 8d may detect hydrogen having a high concentration. Further, the atmosphere in which the catalytic combustion typed gas sensor 8d is placed is estimated to contact with members containing a large amount of the silicone compound in the fuel cell 2a and the accessory 2b. Accordingly, it is estimated that the atmosphere contains a large amount of the silicone compound evaporated from the members.

FIG. 2 shows a cross-sectional drawing of the catalytic combustion typed gas sensor 8 (or 8a to 8d) in an embodiment of the present invention. Herein, sensors having the same structures may be respectively used as the catalytic combustion typed gas sensors 8a to 8d (8) without depending on the hydrogen concentration to be detected or the concentration of the silicone compound contained in the atmosphere to be set.

The catalytic combustion typed gas sensor 8 is equipped with a substrate 12 and a housing 11 that covers the substrate 12. In the substrate 12, totally 4 electrodes composed of 2 pairs of the electrodes 19 are placed as standing thereon at the lower portion of the substrate 12. The electrode 19 penetrates through the housing 11. The detection element 13 is connected between a pair of the electrodes 19 protruding from the housing 11. Similarly, the compensation element 14 is connected between the other pair of the electrodes 19.
The detection element 13 and the compensation element 14 are placed outside the housing 11, and covered with the housing and the detection unit 15. The detection unit 15 has a detection opening 15a. Therefore, leaked hydrogen and the silicone compound contained in the atmosphere are introduced inside the detection unit 15 through the detection opening 15a, thereby to reach the detection element 13. At the detection opening 15a, it may be preferable to arrange a water repelling filter 16 that repels a water drop, and an adsorption filter 17 that includes active carbon or the like for adsorbing the silicone compound. Further, a heater 18 may be arranged inside the detection unit 15 for the purpose of removing water condensed inside the detection unit 15.

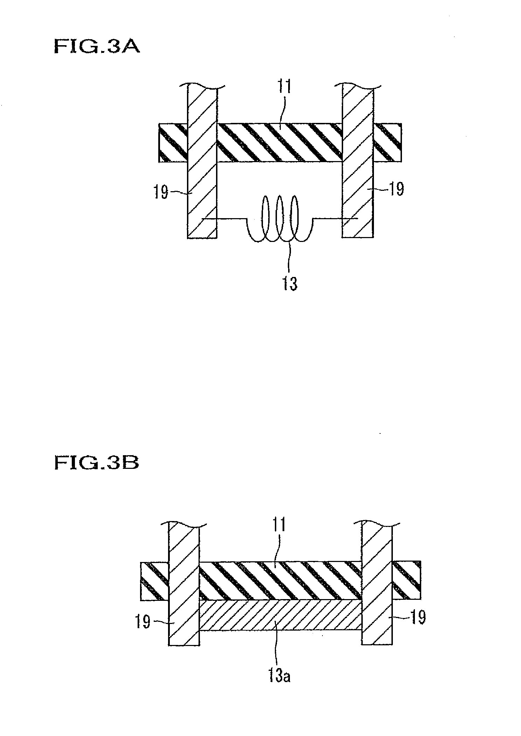

FIG.3A shows a front view of the coil shaped detection element 13. Here, the heater 18 or the like is not shown. In the detection element 13, a wire made of a catalytic metal such as platinum (Pt) or a platinum alloy is formed in a coil shape. The wire of the catalytic metal such as platinum has a catalytic activity that is capable of combusting hydrogen (or combustible gas) present around the wire with oxygen in the air at a low temperature (or conducting the oxidation-reduction reaction). The resulting combustion heat raises the temperature of the detection element 13 (or wire such as platinum), while this makes the heat radiation difficult by forming the detection element 13 (or wire) in a coil shape. This allows the temperature of the detection element 13 (or wire) to be raised higher. Here, the larger the temperature change in the detection element 13 (or wire) becomes, the larger the change in the electric resistance becomes. This allows a highly sensitive detection to be achieved.
On the other hand, the compensation element 14 is constructed by forming the wire such as platinum in a coil shape, of which drawing is omitted. Herein, note a surface of the compensation element 14 made of the wire such as platinum is coated by alumina or the like such that the catalyst becomes inactivated. Accordingly, even though hydrogen (or combustible gas) is present around the wire such as platinum or the like of the compensation element 14, it is not possible to combust hydrogen (or combustible gas) with oxygen in the air (or to conduct the oxidation-reduction reaction). As a result, this construction prevents the compensation element 14 from generating combustion heat, resulting in no change in the electric resistance.

FIG.3B shows a cross-sectional drawing of the detection element 13a having a thin film shape. Here, the heater 18 or the like is not shown. The detection element 13a is made of platinum (Pt) or a thin film of a platinum alloy. It is easy to decrease the thickness of the detection element 13a, if the detection element 13a has a thin film shape. This shape enables a downsizing thereof. Further, if the shape thereof is a thin film like, it is possible to increase the rate of the surface area against the total volume, allowing the temperature of the detection element 13a to be raised up to significantly high due to a large reaction area of the hydrogen (or combustible gas) combustion. Note the compensation element 14 is also constructed in a thin film shape, although the element 14 is not shown in FIG.3B. Herein, the surface of the thin film is coated with aluminum or the like such that the thin film made of platinum or the like in the compensation element 14 becomes catalytically inactive.

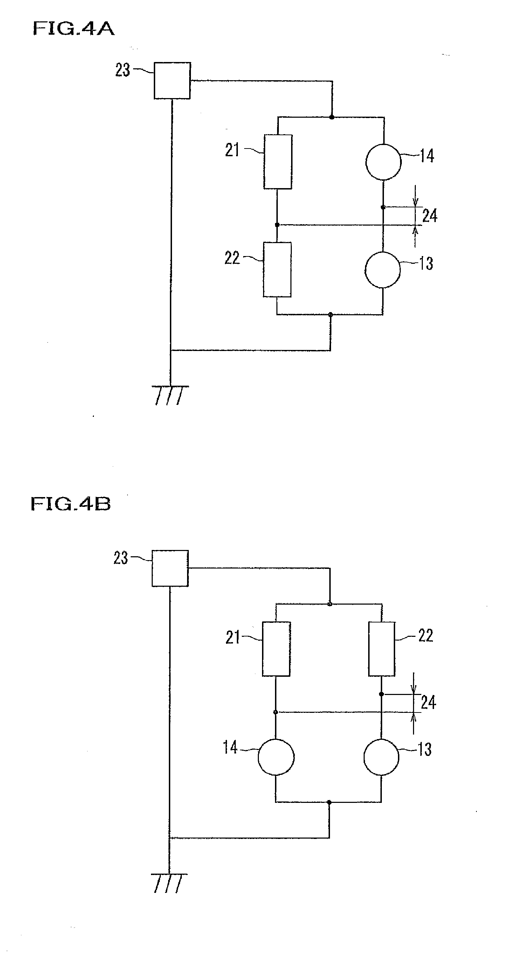

FIG.4A shows a circuit for passing electric current through the elements. In the circuit, the detection element 13 and the compensation element 14 are connected in series. In the circuit for passing electric current through the elements, the detection element 13 and the compensation element 14 are connected in series, and a standard resistor 21 and a standard resistor 22 are connected in series. Further, the detection element 13 and the compensation element 14 connected in series are connected in parallel with the standard resistors 21 and 22. Herein, the connected parts in series as mentioned above are connected to a power source 23.
Here, change in the voltage difference between the voltage of a node connecting to the detection element 13 and the compensation element 14 and the voltage of a node connecting to standard resistors 21 and 22 is shown as a concentration signal 24. The concentration signal 24 represents a detection signal showing how much hydrogen (or combustible gas) has been leaked (that is, detection signal of a raised degree of the hydrogen concentration). If the hydrogen concentration is raised due to the leakage thereof, only the resistance value of the detection element 13 increases by hydrogen combustion. Accordingly, the voltage of the node connecting to the detection element 13 and the compensation element 14 is increased, whereby the concentration signal 24 is output as the change in the voltage difference, allowing the hydrogen leakage to be detected.
For example, if an outside temperature is increased without any hydrogen leakage, the temperature of the detection element 13 and the temperature of the compensation element 14 are raised at a same degree, resulting in the same degree of increases in the resistance values. Therefore, the voltage of the node connecting to the detection element 13 and the compensation element 14 is not changed and the voltage difference is not changed, resulting in no output of the concentration signal 24 indicating the hydrogen leakage.

FIG.4B shows a circuit for passing electric current through the elements, connecting the detection element 13 and the compensation element 14 in parallel. In the circuit for passing electric element through elements, the detection element 13 and the standard resistor 22 are connected in series, and the compensation element 14 and the standard resistor 21 are connected in series. Further, the detection element 13 and the standard resistor 22 connected in series, and the compensation element 14 and the standard resistor 21 connected in series of are connected in parallel. Herein, the power source 23 is connected to the above mentioned units connected in parallel.
Herein, change in the voltage difference between the voltage of a node connecting to the detection element 13 and the standard resistor 22 and the voltage of a node connecting to the compensation element 14 and the standard resistor 21 represents a concentration signal 24. The concentration signal 24 represents a detection signal on how much hydrogen (or combustible gas) has been leaked (that is, detection signal of a raised degree of the hydrogen concentration). If the hydrogen concentration is raised due to the leakage thereof, only the resistance value of the detection element 13 increases by hydrogen combustion. Accordingly, the voltage of the node connecting to the detection element 13 and the standard resistor 22 is increased, whereby the concentration signal 24 is output as the change in the voltage difference, allowing the hydrogen leakage to be detected.
For example, if an outside temperature is increased without any hydrogen leakage, the temperature of the detection element 13 and the temperature of the compensation element 14 are raised at a same degree, resulting in the same degree of increases in the resistance values. Therefore, the voltages applied to the detection element 13 and the compensation element 14 are increased in the same degree, thereby to change no voltage difference. This results in no output of the concentration signal 24 indicating the hydrogen leakage.

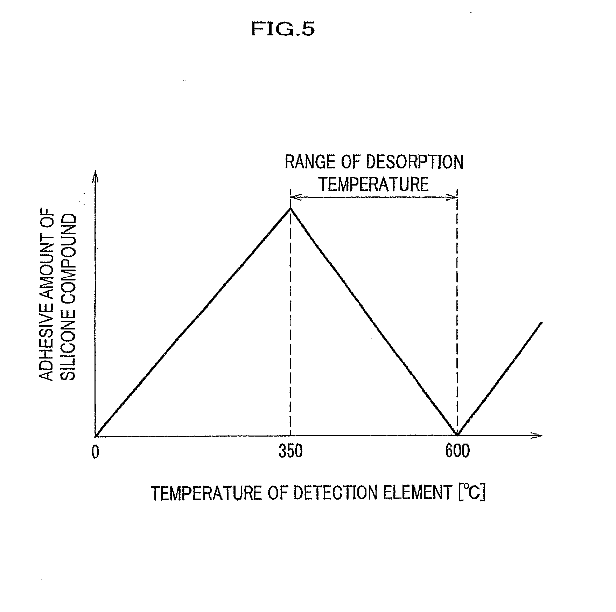

FIG. 5 shows a relationship between the temperature of the detection element 13 and the adhesive amount of the silicone compound (that is, silicon poisoning degree). As generally recognized in the past, it has been considered that the higher the temperature of the detection element 13 is raised, the larger the adhesive amount of the silicone compound (that is, silicon poisoning degree) is increased. However, as shown in FIG.5, if the temperature of the detection element 13 is in the range of the desorption temperature from 350°C to 600°C, the resulting data may indicate that the adhesive amount of the silicone compound (or silicon poisoning degree) is decreased. The reason may be attributed to a phenomenon that the silicone compound once adhered to the detection element 13 is desorbed (or evaporated) from the surface of the detection element 13. In the range of the desorption temperature, the desorption rate becomes substantially to be the same as the order of the adhesive rate of the silicone compound, although the desorption rate may be too slow. That is, the desorption rate is substantially the same as the desorption rate at substantially 600°C. Further, if the temperature becomes more than 600°C, the adhesive rate again becomes faster than the desorption rate.
Moreover, in the range of the desorption temperature from 350°C to 600°C, it has been shown that the higher the temperature of the detection element 13 becomes, the smaller the adhesive amount of the silicone compound becomes.

Furthermore, it has been shown that the temperature rising of the detection element 13 in the desorption temperature range from 350°C to 600°C improved the detection sensitivity (that is, the ratio of the change in the resistance value to the change in the gas concentration). Herein, the improvement tendency of the detection sensitivity is to be increased as the temperature is raised from 350°C to 600°C, and reaches the highest level at 600°C.
In other words, it is preferable to raise the temperature of the detection element 13 up to the desorption temperature in the range from 450°C to 600°C rather than to raise the temperature thereof up to the desorption temperature in the range from 350°C to 600°C. Further, it is more preferable to raise the temperature of the detection element 13 up to the desorption temperature in the range from 500°C to 600°C. Moreover, it is the most preferable to raise the temperature thereof up to the desorption temperature in the range from 550°C to 600°C. Accordingly, it is decided to use the combustion heat when hydrogen (or combustible gas) is detected, in the embodiment of the present invention.

Further, the detection sensitivity may be recovered whenever the fuel cell vehicle 1 and the catalytic combustion typed gas sensor 8 are started up and shut down, if the electric current passes through the detection element 13 at least at the either period as mentioned above such that the temperature of the detection element 13 is to be set in the range from 350°C to 600° at the following period. That period is comprised of at least either of the startup period and the shutdown period of the fuel cell vehicle 1 (see FIG.1), that is, at least either of the startup period and the shutdown period of the catalytic combustion typed gas sensor 8.

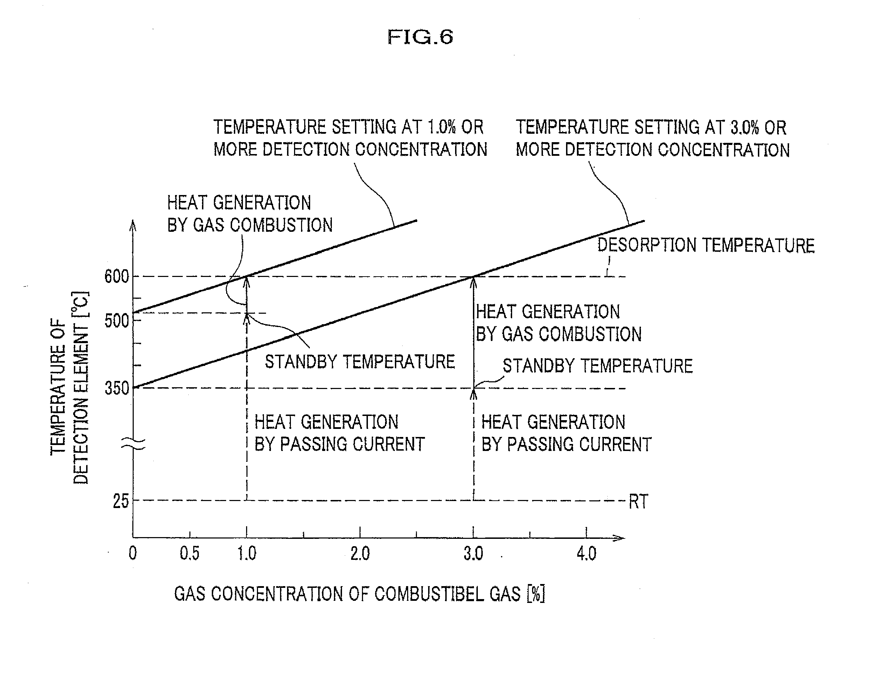

FIG.6 shows a graphic diagram in which the horizontal axis represents gas concentration of the combustible gas (or hydrogen), and the vertical axis represents a temperature of the detection element 13. Herein, the graphic diagram shows a method for setting the desorption temperature and the standby temperature, both in the case that the detection concentration is set at 1.0% (that is, vol%, similarly as described hereinafter) or more, and the case that the detection concentration is set at 3.0% or more.

First, a case that hydrogen is detected if the concentration of the leaked hydrogen becomes 3.0% or more, that is, a case that the detection concentration is set at 3.0% or more will be explained. The detection concentration set at 3.0% allows hydrogen having a high concentration to be detected, enabling the detection element 13 applied to the hydrogen detection inside the off-gas discharge pipe 3 (see FIG.1). Here, the room temperature is defined as 25°C. The detection element 13 and the compensation element 14 perform heat generation by passing electric current therethrough, raising the temperatures thereof up to 350°C. That standby temperature is kept at 350°C until hydrogen leakage occurs. If the hydrogen leakage occurs, the leaked hydrogen undergoes combustion by the detection element 13 thereby to generate heat. Since the concentration of the leaked hydrogen is high at 3.0%, the resultant combustion heat is large, making the raised temperature reach 250°C, different from the initial temperature.
Accordingly, the temperature is raised from the standby temperature of 350°C up to the desorption temperature of 600°C. Since the temperature of the detection element 13 reaches 600°C, the detection sensitivity may be improved. If the detection concentration is set equal to 3.0%, electric current is made to pass through the detection element 13 such that the temperature thereof becomes a standby temperature of 350°C. That temperature is calculated by subtracting the raised temperature portion of 250°C from the desorption temperature of 600°C.
Herein, the raised temperature portion of 250°C is provided by the combustion heat generated by 3.0% hydrogen contacting to the detection element 13. Further, if it is possible to set the desorption temperature in the range from 350°C to 600°C, the standby temperature may be set in the range from 100°C to 350°C, the temperatures being calculated by subtracting the raised temperature portion of 250°C from the above range of the desorption temperatures. Eventually, it is possible to realize both rapid detection and a long life-span of the detection element 13 (or extension of the life-span thereof).

Next, a case that hydrogen is detected if the concentration of the leaked hydrogen becomes 1.0% or more, that is, a case that the detection concentration is set at 1.0% or more will be explained. The detection concentration equal to 1.0% allows the detection of hydrogen at a low concentration, enabling the detection element 13 applied to the hydrogen detection inside a cabin 4 (see FIG.1), of a floor panel 5, or especially of a room below a center console 5a. Here, the room temperature is defined as 25°C. The detection element 13 and the compensation element 14 are made to generate heat by passing electric current therethrough until each temperature thereof is raised up to around 520°C. Then, the detection element 13 and compensation element 14 are kept at the standby temperature of around 520°C until hydrogen leakage occurs.
Once hydrogen leaks, the leaked hydrogen is combusted by the detection element 13 to generate heat. The low concentration in 1.0% of the leaked hydrogen generates a small quantity of heat. The raised temperature portion is around 80°C, whereby the temperature is raised up to in the range from around 520°C to 600°C. Accordingly, the temperature of the detection element 13 reaches 600°C, allowing the detection sensitivity to be improved. If the detection concentration is set at 1.0%, electric current is made to pass through the detection element 13 such that the standby temperature becomes around 520°C which is calculated by subtracting the raised temperature portion of 80°C generated by the combustion heat from the desorption temperature of 600°C.
Herein, the combustion heat is generated when hydrogen having a detectable concentration of 1.0% is combusted by contacting with the detection element 13. If the desorption temperature is set in the range from 350°C to 600°C, it is possible to set the standby temperature in the range from around 270°C to around 520°C, of which values may be calculated by subtracting the raised temperature portion of around 80°C from the temperatures in the range from 350°C to 600°C. This setting enables both rapid detection and a long life-span of the detection element 13 (or extension of the life-span thereof) to be realized.

Further, according to the procedure as described above, in contrast, if the standby temperature is set in the range from 350°C or more to less than 600°C, it is possible to set the detection concentration in the range of 3.0& or less and the desorption temperature to be at 600°C.

### DESCRIPTION OF REFERENCE NUMERALS

1 Fuel Cell Vehicle
2 Fuel Cell System
2a Fuel Cell Battery
2b Accessory
3 Off-Gas Discharge Pipe
4 Cabin
4a Cabin Top
5 Floor Panel
5a Center Console
6 Hydrogen Tank
7 Hydrogen Supply Pipe
8, 8a, 8b, 8c, 8d Catalytic Combustion Typed Gas Sensor
11 Housing
12 Substrate
13, 13a detection Element
14 Compensation Element
15 Detection Unit
15a Detection Opening
16 Repelling Filter
17 Adsorption Filter
18 Heater
19 Electrode
21, 22 Standard Resistor
23 Power Source
24 Concentration Signal

## Claims

1. A catalytic combustion typed gas sensor comprising a catalytic metal, wherein
a temperature and electric resistance of the catalytic metal are raised when a combustible gas contacts to the catalytic metal heated by passing electric current through the catalytic metal so as to undergo combustion, and the resulting raised electric resistance of the catalytic metal is applied to detection of the combustible gas having a concentration equal to or more than a predetermined concentration, and
electric current is made to pass through the catalytic metal such that the temperature of the catalytic metal becomes a standby temperature which is calculated by subtracting a raised temperature portion from a desorption temperature; the raised temperature portion being generated by combustion heat when the combustible gas having the predetermined concentration contacts to the catalytic metal; the catalytic metal adsorbing a silicone compound via a silicon (Si) poisoning process and then desorbing the resulting adsorbed silicone compound at the desorption temperature.

2. The catalytic combustion typed gas sensor as described in claim 1, wherein the desorption temperature is set in the range from a temperature over 350°C to 600°C or less.

3. The catalytic combustion typed gas sensor as described in claim 1, wherein
the catalytic combustion typed gas sensor is arranged in an atmosphere; a silicone compound concentration contained in the atmosphere being higher than in the air, and
the standby temperature is set in the range from 100°C or more to 350°C or less.

4. The catalytic combustion typed gas sensor as described in claim 1, wherein
the catalytic combustion typed gas sensor is arranged in the air, and
the standby temperature is set in the range from 270°C or more to 520°C or less.

5. The catalytic combustion typed gas sensor as described in any one of claims 1 to 4, wherein electric current is made to pass through the catalytic metal such that a temperature of the catalytic metal becomes in the range from 350°C to 600°C at a time of at least either a startup period and a shutdown period.

6. The catalytic combustion typed gas sensor as described in claim 1, wherein the catalytic combustion typed gas sensor is arranged inside an off-gas discharge pipe through which air supplied to a cathode of a fuel cell is discharged.

7. The catalytic combustion typed gas sensor as described in claim 1, wherein a fuel cell vehicle is equipped with the catalytic combustion typed gas sensor.
